(19) Europäisches Patentamt
European Patent Office
Office européen des brevets

(11) EP 2 070 938 A1

(12) EUROPEAN PATENT APPLICATION

(43) Date of publication:
**17.06.2009 Bulletin 2009/25**

(51) Int Cl.:
***C07H 19/20*** *(2006.01)* ***A61K 31/7076*** *(2006.01)*

(21) Application number: **07024198.9**

(22) Date of filing: **13.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Heidelberg Pharma AG**
**69115 Heidelberg (DE)**

(72) Inventor: **The designation of the inventor has not yet been filed**

# (54) Clofarabine dietherphospholipid derivatives

(57) The subject of the present invention are specific phospholipidesters of clofarabine and the use of such lipidesters in the treatment of tumors.

EP 2 070 938 A1

**Description**

The subject of the present invention are specific phospholipidesters of clofarabine of the general formula I,

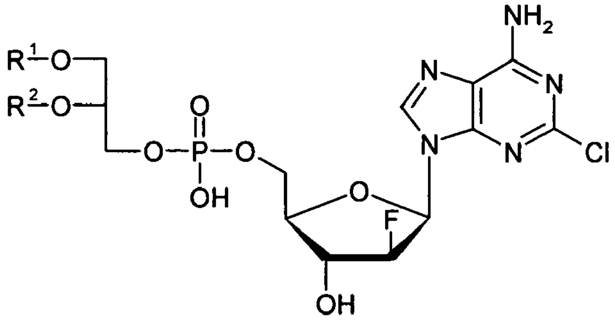

(I)

wherein

$R^1$ and $R^2$ are independently selected from the group consisting of a straight-chain or branched, saturated or unsaturated alkyl chain having 1-20 carbon atoms,
their tautomers and their physiologically acceptable salts of inorganic and organic acids and bases, as well as processes for their preparation and medicaments containing these compounds as active ingredients.

Since the compounds of the general formula I contain asymmetric carbon atoms, all optically-active forms, including single diastereomeric forms, mixtures thereof and racemic mixtures of these compounds are also the subject of the present invention.

J. Biol. Chem. 265, 6112-6117 (1990) and EP-A-0,350,287 describe preparation and use of phospholipidesters of nucleosides as anti-viral drugs. Therein, however, only dimyristoylphosphatidyl and dipalmitoylphosphatidyl residues coupled to well known nucleosides such as AZT and DDC are disclosed, including their fatty acid ester structure.

In US 5.512.671 conjugates of ether lipids and antiviral nucleoside analogues are disclosed having an amide alkyl glycerol residue as lipid moiety.

The patent application WO 92/03462 describes ether lipid conjugates with other nucleosides than clofarabine having antiviral activity, particularly for the treatment of HIV infections, preferably thioether lipids

The patent application EP-A-1 460 082 describes thioether lipid conjugates suitable for therapy and prophylaxis of malignant tumors including, carcinomas, sarcomas, or leukemias, however only thioethergycerol derivatives, having the thioethergroup in position 3 where exemplified

The synthesis of 2-Chloro-9-(2'-deoxy-2'-fluoro-β-D-arabinofuranosyl)adenine (clofarabine) is described in J. Org. Chem. 34, 2632 -2636 (1969), in patent application WO 01/60383, and in U.S. patent 6,680,382.

The pharmacological activity of clofarabine as inhibitor of DNA replication in comparison to other nucleosides is also described in Hematology 463 (1999).

Clofarabine is approved for pediatric acute lymphoblastic leukemia (ALL.

The compounds of the present invention of general formula I posses biological activity which distinguish them from the parent nucleoside and the thioether lipid conjugates described in patent application EP-A-1 460 082. Surprisingly it has been found that the compounds of the present invention exert a stronger and longer lasting anti-tumoral activity in comparison with the thioether lipid conjugates exemplified in EP-A-1 460 082. One reason for this observation may be the difference with regard to their property to be oxidized. Thioether lipid conjugates are in contrast to dioxyether lipid conjugates highly sensitive to oxygen. Chemical pure as well as pharmaceutical forms of thioether lipid conjugates become oxidized to their sulfoxide derivatives over time. Even more important a certain proportion of thioether lipid conjugates are metabolically oxidized to their sulfoxides once they have entered the body. The pharmacological disadvantage of sulfoxidized lipid conjugates is their very short a half life of less than 20 minutes. They are eliminated very fast by the liver via the gull bladder into the intestine. By comparison the known thioether lipid conjugates as well as the dioxyether lipid conjugates of the present application have a plasma half life of more than 6 hours. As a result, the more thioether lipid conjugate is oxidized to sulfoxidized lipid conjugate the less of the thioetherlipid conjugate is available to act on the tumor. In contrast dioxyether lipid conjugates can not be oxidized chemically or metabolically. Therefore no fast elimination of oxidized forms of the conjugate will occur. As a consequence their full pharmacological activity comes into operation.

Chemotherapeutic agents differ greatly with regard to their distribution volume i.e their property to distribute into the tissues of the body. Chemotherapeutic drugs must come in contact with the tumor cells to exert their cytotoxic activity. Bioanalytical studies surprisingly revealed that with dioxyether lipd conjugates the distribution volume is significantly higher than that of thioether lipid conjugates. As a result tumors will be exposed to a higher concentrations of the drug. This unexpected difference in body distribution of dioxyether-conjugates compared with thioetherlipid conjugates is a pharmacological relevant quality resulting in a superior anti tumor activity. The better tissue penetration of dioxyether-conjugates contributes to their enhanced selectivity and effectiveness finally providing a wider therapeutic window.
The chemical stability and the favourable body distribution of the dioxyether lipid conjugates in contrast to thioetherlipid conjugates are responsible for the fact that the compounds of the present invention are especially suitable for therapy and prophylaxis of malignant tumors including, carcinomas, sarcomas, or leukemias.

In some embodiments of the present invention, the administration of pharmaceutical compositions comprising these compounds may be conducted continuously over a prolonged period of time. Incidences of withdrawal of the preparation or intermittent administration, which frequently are routine with chemotherapeutic agents due to their undesirable side-effects, may be reduced with the compounds according to this invention as compared to the parent compounds and thioether lipid conjugates. Further, higher dose levels may be employed due to the amelioration of toxic side effects due to enhanced selectivity for tumor cytotoxicity.

The lipidphosphoester compounds of the present invention are also suitable for the treatment of autoimmune disorders, including multiple sclerosis, rheumatoid arthritis, lupus, systemic vasculitis, inflammatory bowel disease, scleroderma and Sjorgen's syndrome.

The lecithin-like structure of the lipid moiety is desirable for the claimed improvements of the compounds of general formula I. The penetration through membranes and reabsorption barriers is facilitated and the conjugates according to formula I show a depository effect in different tissues.

The formation of lipid conjugates may also facilitate crossing the blood brain barrier due to better diffusion or active transport processes.

Similarly, the compounds of the present invention and their pharmaceutical formulations may be employed in free or fixed combination with other drugs for the treatment and prophylaxis of the diseases mentioned above.
Examples of these further drugs involve agents such as, e.g., mitosis inhibitors such as colchicines, vinblastine, alkylating cytostatic agents such as cyclophosphamide, melphalan, myleran or cis-platin, antimetabolites such as folic acid antagonists (methotrexate) and antagonists of purine and pyrimidine bases (mercaptopurine, 5-fluorouridine, cytarabine), cytostatically active antibiotics such as anthracyclines (e.g., doxorubicin, daunorubicin), hormones such as fosfestrol, tamoxifen, taxanes, e.g. taxol, and other cytostatically/cytotoxically active chemotherapeutic and biologic agents.

Embodiments of the invention also encompass salts of the compounds of the general formula I, including alkali, alkaline earth and ammonium salts of the phosphate group. Examples of the alkali salts include lithium, sodium and potassium salts. Alkaline earth salts include magnesium and calcium and ammonium salts are understood to be those containing the ammonium ion, which may be substituted up to four times by alkyl residues having 1-4 carbon atoms, and/or aryl residues such as benzyl residues. In such cases, the substituents may be the same or different.

The compounds of general formula I may contain basic groups, particularly amino groups, which may be converted to acid addition salts by suitable inorganic or organic acids. To this end, possible as the acids are, in particular: hydrochloric acid, hydrobromic acid, sulphuric acid, phosphoric acid, fumaric acid, succinic acid, tartaric acid, citric acid, lactic acid, maleic acid or methanesulfonic acid.

In general formula I, $R^1$ preferably represents a straight-chain $C_8$-$C_{16}$ alkyl residue. More specifically, $R^1$ represents an octyl, nonyl, decyl, undecyl, dodecyl residue. Preferably, $R^2$ represents a straight-chain $C_8$-$C_{16}$ alkyl group. More specifically, $R^2$ represents an octyl, nonyl, decyl, undecyl, dodecyl group.
An example of a preferred lipid moiety is the group

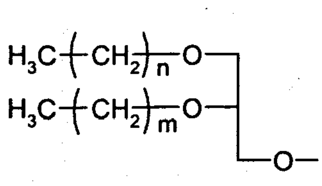

wherein n and m are independently an integer from 10 to 12

In detail the the moiety is:

- (2-decyloxy-3-dodecyloxy)propyl-

- (2-decyloxy-3-undecyloxy)propyl-
- 2,3-didecyloxy)propyl-
- (3-dodecyloxy-2-undecyloxy)propyl-
- (2,3-diundecyloxy)propyl-
- (3-decyloxy-2-undecyloxy)propyl-
- (2,3-didodecyloxy)propyl-
- (2-dodecyloxy-3-undecyloxy)propyl-
- (3-decyloxy-2-dodecyloxy)propyl-

group

The compounds of the general formula I are prepared by reacting a compound of general formula II, or a salt form thereof,

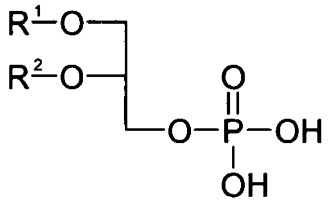

(II)

wherein $R^1$ and $R^2$ have the meaning as indicated above, with clofarabine of formula III

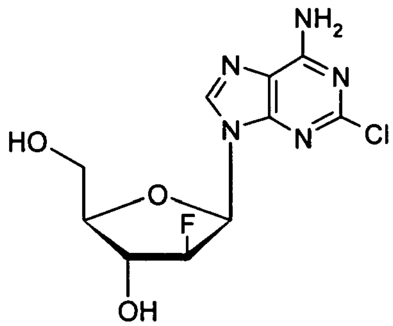

(III)

wherein the 3'-hydroxy group may optionally be protected by an oxygen protecting group familiar to the artisan, and the compound of formula II may be activated in the presence of an appropriate- acid chloride, such as 2,4,6-triisopropylbenzenesulfonic chloride, and a tertiary nitrogen base, e.g., pyridine or lutidine, in an inert solvent, such as toluene, or immediately in anhydrous pyridine, and optionally, subsequent to hydrolysis, removing the oxygen protecting groups according to procedures conventional in nucleoside chemistry.

or

reacting a lipidalcohol (corresponding to formula II) with a nucleoside-5'-monophosphate (corresponding to formula III) in the same manner as mentioned above.

The preparation of the compounds of the general formula II is performed in analogy to WO 96/06620 and DE 3934820 Clofarabine of formula III is prepared in analogy to J. Org. Chem. 34, 2632 -2636 (1969), J. Med. Chem. 35, 397 - 401 (1992) or WO 01/ The first step comprises the preparation of 2,6-dichloro-9-(3', 5'-O-dibenzoy)-2'-deoxy-2'-fluoro-β-D-arabinofuranosyl)-9*H*-purine by reacting 2,6-dichloropurine with a blocked 2-deoxy-2-fluoro-α-D-arabinofuranosyl halide in a suitable solvent in the presence of a hindered potassium base, preferably potassium *t*-butoxide or potassium *t*-amylate. Suitable blocking groups include benzoyl and acetyl. Suitable halides include bromide and chloride. Suitable inert solvents include, but are not limited to, *t*-butyl alcohol, acetonitrile, dichloromethane, dichloroethane, *t*-amyl alcohol, tetrahydrofuran or mixtures thereof. A preferred solvent comprises a mixture of acetonitrile, *t*-butanol and 1,2-dichloroethane. Calcium hydride may be optionally added to the reaction mixture. The second step comprises subjecting the 2,6-dichloropurine nucleoside derivative to conditions that provide for deprotection and an aromatic nucleophilic substitution reaction, e.g., sodium hydroxide and $C_1$-$C_8$ alcohol or sodium $C_1$-$C_8$ alkoxide in the corresponding $C_1$-$C_8$ alcohol

(e.g., methanol with sodium methoxide, ethanol with sodium ethoxide, etc.) or other suitable nonalcoholic solvent, to provide a $C_1$-$C_8$ 6-alkoxy purine nuceloside and subsequent reaction of this intermediate with anhydrous ammonia in a $C_1$-$C_8$ alcohol or other suitable solvents, or direct exposure of the 2,6-dichloropurine nucleoside to anhydrous ammonia in a $C_1$-$C_6$-alcohol to provide the clofarabine of formula III.

Salts of compounds of general formula I are prepared by reacting the free acid with alkali or alkaline earth hydroxides, alcoholates or acetates.

The "enantiomers" in the lipid parts of the compounds of formula I may be prepared by separation via diastereomeric salts or by enantioselective synthesis of the lipid residues starting with optically active $C_3$ -precursors of formula II, like (+)-or (-)-isopropylidinegycerol by methods disclosed in WO 96/06620 and EP 0315973.

The drugs containing compounds of formula I for the treatment of cancer may be administered in liquid or solid forms on the oral or parenteral route. Common application forms are possible, such as tablets, capsules, coated tablets, syrups, solutions, or suspensions.

Preferably, water is used as the injection medium, containing additives such as stabilizers, solubilizers and buffers as are common with injection solutions. Such additives are, e.g., tartrate and citrate buffers, ethanol, complexing agents such as ethylenediaminetetraacetic acid and its non-toxic salts, high-molecular polymers such as liquid polyethylene oxide for viscosity control. Liquid vehicles for injection solution need to be sterile and are filled in ampoules, preferably. Solid carriers are, for example, starch, lactose, mannitol, methylcellulose, talc, highly dispersed silicic acids, higher-molecular fatty acids such as stearic acid, gelatine, agar-agar, calcium phosphate, magnesium stearate, animal and plant fats, solid high-molecular polymers such as polyethylene glycol, etc. If desired, formulations suitable for oral application may include flavorings or sweeteners.

The dosage may depend on various factors such as mode of application, species, age, or individual condition.

The compounds according to the invention may suitably be administered orally or intravenously (i.v.) in amounts in the range of 0.1 - 100mg, preferably in the range of 0.2 - 80mg per kg of body weight and per day. In some dosage regimens, the daily dose is divided into 2-5 applications, with tablets having an active ingredient content in the range of 0.5 - 500mg being administered with each application.

Similarly, the tablets may have sustained release, reducing the number of applications, e.g., to 1-3 per day. The active ingredient content of sustained-release tablets may be in the range of 2-1000mg. The active ingredient may also be administered by i.v. bolus injection or continuous infusion, where amounts in the range of 5-1000mg per day are normally sufficient.

In addition to the compounds mentioned in the examples, the following compounds of formula I, their optically-active forms, including single diastereomeric forms, mixtures thereof and their pharmacologically acceptable salts further exemplify compounds of the present invention:

1. [2-Chloro-9-(2'-deoxy-2'-fluoro-β-D-arabinofuranosyl)adenine]-5'-phosphoric acid-(2-decyloxy-3-undecyloxy) propyl ester
2. [2-Chloro-9-(2'-deoxy-2'-fluoro-β-D-arabinofuranosyl)adenine]-5'-phosphoric acid-(3-dodecyloxy-2-undecyloxy) propyl ester
3. [2-Chloro-9-(2'-deoxy-2'-fluoro-β-D-arabinofuranosyl)adenine]-5'-phosphoric acid-(2,3-diundecyloxy)propyl ester
4. [2-Chloro-9-(2'-deoxy-2'-fluoro-β-D-arabinofuranosyl)adenine]-5'-phosphoric acid-(3-decyloxy-2-undecyloxy) propyl ester
5. [2-Chloro-9-(2'-deoxy-2'-fluoro-β-D-arabinofuranosyl)adenine]-5'-phosphoric acid-(2-dodecyloxy-3-undecyloxy) propyl ester
6. [2-Chloro-9-(2'-deoxy-2'-fluoro-β-D-arabinofuranosyl)adenine]-5'-phosphoric acid-(3-decyloxy-2-dodecyloxy) propyl ester

**Example 1**

**Preparation of [2-Chloro-9-(2'-deoxy-2'-fluoro-β-D-arabinofuranosyl)adenine]-5'-phosphoric acid-(2-decyloxy 3-dodecyloxy)propyl ester (mixture of diastereomers) (1)**

I

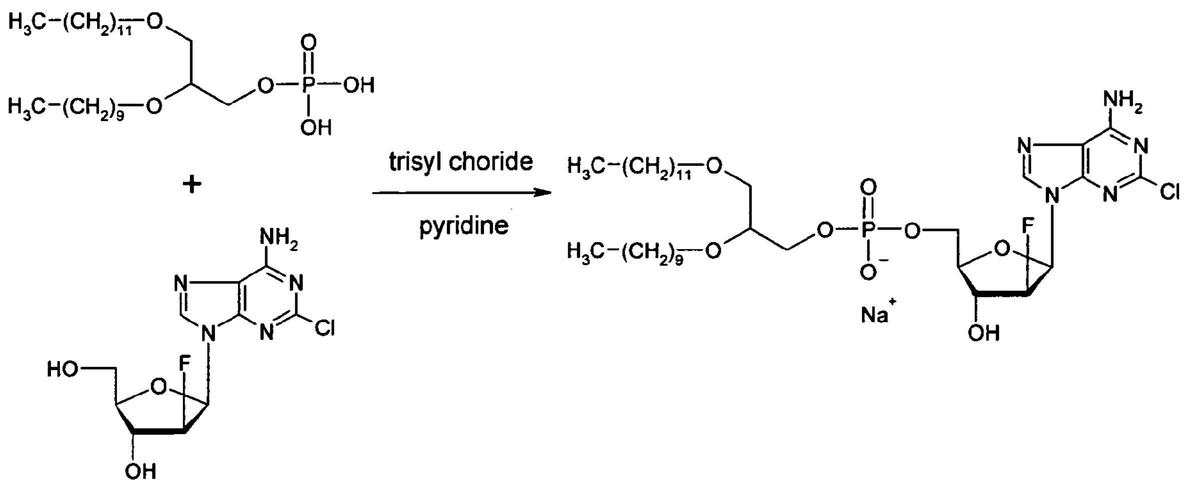


4,31 g (8,97 mmol) of rac-phosphoric acid-(3-dodecyloxy-2-decyloxy)propyl ester are treated twice with 50 ml of anhydrous pyridine and concentrated by evaporation. The residue is dissolved in 180 ml of anhydrous pyridine at room temperature, treated with 5,43 g (17,93 mmol = 2 eq.) of 2,4,6-triisopropylbenzenesulfonyl chloride (trisyl choride) under nitrogen and stirred at 20-25°C for 1 hour. Then 4,08 g (13,45 mmol = 1,5 eq.) of 2-chloro-9-(2'-deoxy-2'-fluoro arabinofuranosyl)adenine (Clofarabine) are added at once, and the mixture is stirred under nitrogen for 20 hours. Hydrolysis is performed by adding 100 ml of saturated sodium hydrogencarbonate solution, the mixture is stirred for another 0.5 hour at room temperature until ending of carbon dioxide evolution and freed from solvent under vacuum at 40°C. Acetone (150 ml) is added to the solid residue vigorously stirred for an additional hour. The resulted suspension is filtrated with suction and the residual salt is washed with 50 ml acetone. The combined acetone filtrate and washings are concentrated and the residue is purified by HPLC on Lichrospher 60 RPSelect B with methanol/aqueous 40mM sodium acetate 85: 15 as the eluent. The product containing fractions are evaporated. The residue is distributed between 200 ml of tert.-butylmethylether and 60 ml of 2N hydrochloric acid. The organic layer is evaporated, the residue is dissolved in a mixture of 20 ml of methanol and the pH is adjusted to pH 7 by addition of sodium methanolate (30 % in methanol). The solvent is stripped of and the residual sodium salt dissolved in 25 ml acetone and dropped at 0°C to 50 ml acetonitrile. The resulting suspension is stirred for 1 h at 0 °C , the precipitate is filtered off with suction washed with 50 ml ice cold acetonitrile and dried in vacuum: 4,66 g (66 %) sodium salt of 1 as white amorphous powder.

$^1$H NMR (300 MHz, DMSO-$d_6$): 8.2 (s, 1 H, $H_8$), 8,0, (s (br), 1 H, $NH_2$), 6,6, (s, 1 H, 3'-OH), 6.3 (dd, 1 H, $H_{1'}$), 5.2 (dt, 1H, $H_{2'}$), 4.5, (dt, 1 H, $H_{3'}$), 3.9-4.0, (m, 3H, $H_{4'}$, $POC\underline{H}_2$), 3.6, (m, 1 H, -$H_{5a'}$), 3.6 (m, 1 H, $H_{5'b}$), 3.2-3.5 (m, 7H, >$C\underline{H}OC\underline{H}_2$-, - $C\underline{H}_2OC\underline{H}_2$), 1.1-1.4 (m, 32H, -$(C\underline{H}_2)_9$-, -$(C\underline{H}_2)_7$-), 0.8 (m, 6H, $CH_2$-$C\underline{H}_3$); $^3J_{1'\text{-H},2\text{-H}} \approx {}^3J_{2'\text{-H},3'\text{-H}} \approx {}^3J_{3'\text{-H},4'\text{-H}} \approx 4.6$ Hz, $^3J_{1'\text{-H,F}} = 13.2$ Hz, $^2J_{2'\text{-H,F}} = 52,7$ Hz, $^3J_{3'\text{-H,F}} = 19.4$ Hz. $^{13}$C NMR (75,0 MHz, DMSO-$d_6$): 156.6, 153.1, 150.0 (C-2, C-4, C-6), 139.7 (C-8), 117.2 (C-5), 95.8+ 93.9 (C-2'), 82,0 (C-4'), 81.2 (C-1'), 77,7 (O-$\underline{C}$H<), 73,1 (C-3'), 70.7, 70.4 ($CH_2$-$\underline{C}H_2O$-$CH_2$-), 69.1 ($CH_2$-$\underline{C}H_2O$-CH<), 63.6 (C-5'), 63.3 (5'-OP(O)$_2$OC$\underline{H}_2$), 21,8-31,1 (-$(CH_2)_9$-, -$(CH_2)_7$-), 13.6 (2× $CH_3$)

$^{31}$P NMR (121,5 MHz, DMSO-$d_6$): 0,23 ppm (singlet).

$^{19}$F NMR (282 MHz, , DMSO-$d_6$): -198.5, -199,5 ppm (2 singlets -1H-decoupled). Mass spec. (FAB$^-$): m/z = 764.39 [M-$Na^+$],

Optical rotation: $[\alpha]_D^{20} = +20.6\,°$ (c = 1.0 in methanol)

The phosphoric acid-(3-dodecyloxy-2-decyloxy)propyl ester is prepared as described in WO 96/06620.

**Example 2**

**Preparation of [2-Chloro-9-(2-deoxy-2'-fluoro-β-D-arabinofuranosyl)adenine]-5'-phosphoric acid-(2*R*-decyloxy 3-dodecyloxy)propyl ester (2)**

By using the method of Example 1 starting from enantiomerically pure phosphoric acid-(2R-decyloxy-3-dodecyoxo-)propyl ester (synthesised from (S)-(+)-1,2-Isopropylideneglycerol by the method disclosed in WO 96/06620) the title compound 2 is received as sodium salt in 76 % yield.
$^{19}F$ NMR (282 MHz, , DMSO-$d_6$): -199.5 ppm (singlet -1 H-decoupled).

Optical rotation: $[\alpha]_D^{20} = +25.1°$ (c = 1.0 in methanol)

**Example 3**

**Preparation of [2-Chloro-9-(2'-deoxy-2'-fluoro-β-D-arabinofuranosyl)adenine]-5'-phosphoric acid-(2*S*-decyloxy 3-dodecyloxy)propyl ester (3)**

By using the method of Example 1 starting from enantiomerically pure phosphoric acid-(2S-decyloxy-3-dodecyloxoy)propyl ester (synthesised from (R)-(-)-1,2-Isopropylideneglycerol by the method disclosed in WO 96/06620) the title compound **3** is received as sodium salt in 28 % yield.

$^{19}F$ NMR (282 MHz, , DMSO-$d_6$): -198.5 ppm (singlet -1 H-decoupled). Optical rotation: $[\alpha]_D^{20} = +17.1°$ (c = 1.0 in methanol)

**Example 4**

**Preparation of [2-Chloro-9-(2'-deoxy-2'-fluoro-β-D-arabinofuranosyl)adenine]-5'-phosphoric acid (2,3-didodecyloxy)propyl ester (4)**

The synthesis is performed according to the method described in example 1 starting from phosphoric acid-(2,3-didodecyloxy)propyl ester
Yield 57 %.
Mass spec. ($FAB^-$): m/z = 792,42 [$M-Na^+$],

**Example 5**

**Preparation of [2-Chloro-9-(2'-deoxy-2'-fluoro-β-D-arabinofuranosyl)adenine]-5'-phosphoric acid (2,3-didecyloxy)propyl ester (5)**

The synthesis is performed according to the method described in example 1 starting from phosphoric acid-(2,3-didecyloxy)propyl ester
Yield 48 %.
Mass spec. ($FAB^-$): m/z = 736.36 [$M-Na^+$],
**[0031]** The phosphoric acid-(2,3-didodecyloxy)propyl ester and phosphoric acid-(2,3-didecyloxy)propyl ester are analogously prepared as described in WO 96/06620, whereas the corresponding lipid alcohols are synthesized by the method disclosed in EP 315973.

**EXAMPLE 6**

**Tablet formulation**

1.50 kg [2-Chloro-9-(2'-deoxy-2'-fluoro-β-D-arabinofuranosyl)adenine]-5'-phosphoric acid-(2-decyloxy-3-dodecyloxy)propyl ester sodium salt,
1.42 kg microcrystalline cellulose,
1.84 kg lactose,
0.04 kg Polyvinylprrolidine and

0.20 kg magnesium stearate
were mixed in dry form, moistened with water and granulated. After drying tablets of 200 mg weight were produced

**EXAMPLE 7**

**Formulation for injection**

10.0 g [2-Chloro-9-(2'-deoxy-2'-fluoro-β-D-arabinofuranosyl)adenine]-5'-phosphoric acid-(2-decyloxy-3-dodecyloxy)propyl ester sodium salt were dissolved in 500 ml physiologic sodium chloride solution, filled at 5 ml in ampoules and sterilized. The solution may be applied by intravenous injection.

**Example 8**

**Antitumor activity of 2-Chloro-9-(2'-deoxy-2'-fluoro-β-D-arabinofuranosyl)adenine]-5'- phosphoric acid-(2-decyloxy 3-dodecyloxy)propyl ester (1) and 2-Chloro-9-(2'-deoxy-2'-fluoro-β-D-arabinofuranosyl)adenine]-5'-phosphoric acid-(2-decyloxy-3-dodecylmercapto)propyl ester(6)in a human cervix carcinoma xenograft model (KB-3-1) in vivo**

The antitumor activity of **1** and the thioether derivative **6,** disclosed in EP-A-1 460 082 has been compared in the human cervix carcinoma xenograft KB-3-1 model in nude mice.
Tumor bearing mice were randomized on day 8 after KB-3-1 tumor cell inoculation and were distributed to treatment groups of n = 7 animals per group. Treatment was started on day 8. The animals were treated orally once daily for 5 consecutive days with **1** or **6.** Dosages included 50 and 25 % of the Maximum Tolerable Doses (MTD's). Control animals received the vehicle (water). On day 30, the primary tumors were explanted and the tumor weights were determined. The median tumor weights are shown in Table1. Animals treated with **1** have significantly ($p < 0.01$) smaller tumors than control animals even 18 days after termination of treatment.

**Table1**

| Tumor weight after treatment with vehicle, **1** or **6** | | | | |
|---|---|---|---|---|
| Compound | MTD | Dose (mg/kg/day) | Tumor weight (g) | Tumor inhibition (%) |
| Control (Vehicle) | - | 0 | 2.79 | |
| **6*** | 25 % | 100 | 1.91 | 31 |
| **6*** | 50 % | 200 | 0.73 | 74 |
| **1** | 25 % | 100 | 1.60 | 43 |
| **1** | 50 % | 200 | 0.19 | 93 |
| *2-Chloro-9-(2'-deoxy-2'-fluoro-β-D arabinofuranosyl)adenine]-5'-phosphoric acid-(3-dodecylmercapto-2-decyloxy)propyl ester is prepared as disclosed in EP-A-1 460 082 | | | | |

**Example 9**

**Oral bioavailability and distribution of 2-Chloro-9-(2'-deoxy-2'-fluoro-β-D-arabinofuranosyl)adenine]-5'- phosphoric acid-(2-decyloxy 3-dodecyloxy)propyl ester (1) and 2-Chloro-9-(2'-deoxy-2'-fluoro-β-D-arabinofuranosyl)adenine]-5'-phosphoric acid-(2-decyloxy-3-dodecylmercapto)propyl ester(6) in mice**

The oral bioavailability and distribution of 1 and the thioether derivative 6, disclosed in EP-A-1 460 082 has been determined in mice.
Mice received 30mg/Kg of 1 or 6 intravenously or orally. For each time point blood of 3 mice has been collected, plasma prepared and the concentration of 1 or 6 has been determined by mass spectroscopy. Blood samples have been taken at 0, 30min, 1,2,3,4,5,7,16 and 24 hours after application of the drug. Bioavailability and the area under the curve (AUC (0-t) [μg h/L] = AUC) have been determined.
As shown in Fig. 1 the oral bioavailability was 28.5% and 31.8% for 1 and 6 respectively. The AUC after oral application was 88927μg/h/L for 1 and 212685 μg/h/L for 6. The distribution of 1 was by the factor 2.4 significantly higher than that of 6.

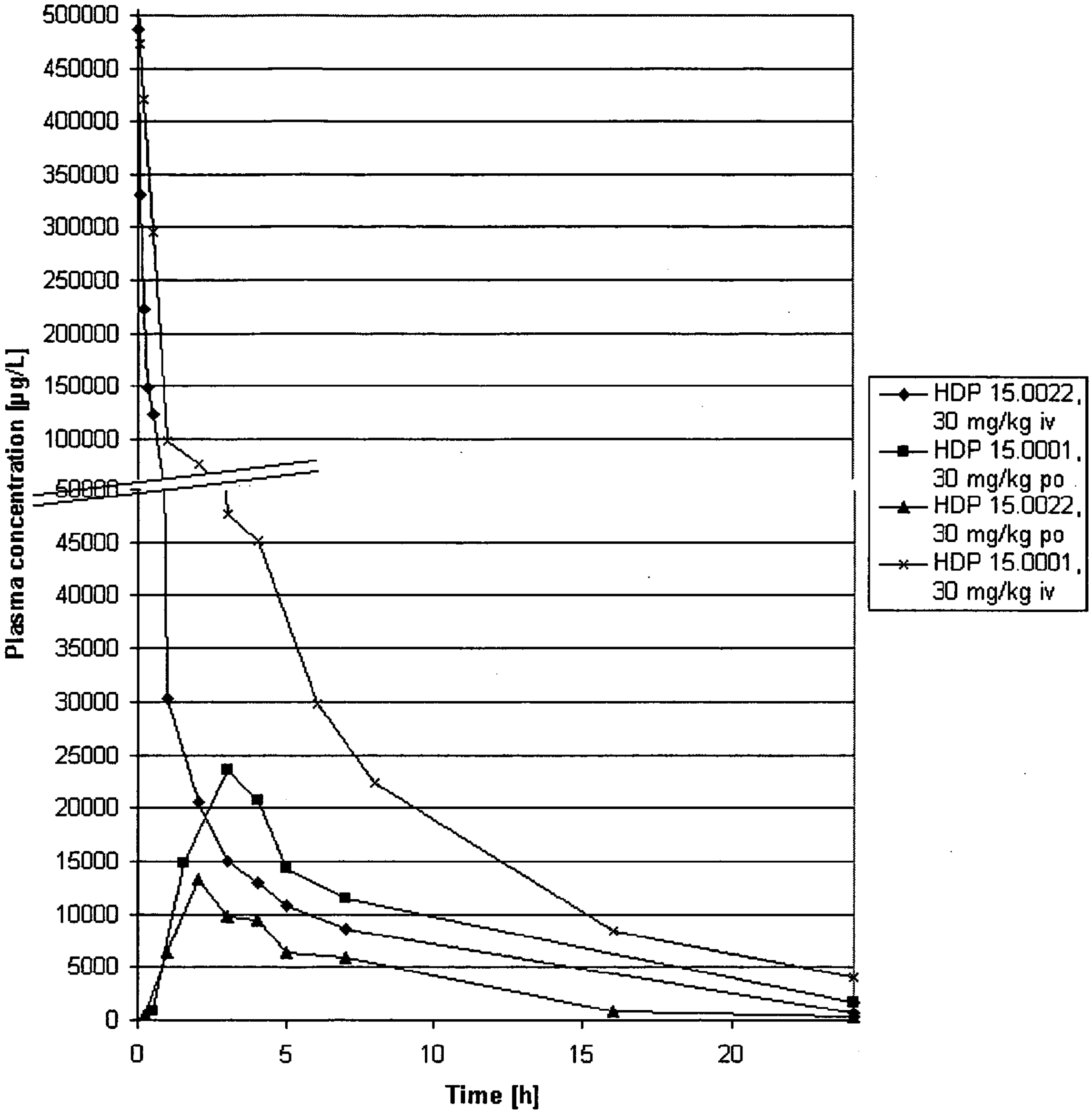


Fig.1

**Claims**

1. A nucleotide derivative of formula I

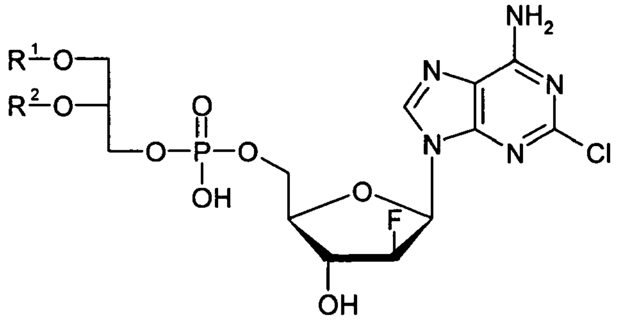

(I)

wherein

R¹ and R² are independently selected from the group consisting of a straight-chain or branched, saturated or unsaturated alkyl chain having 1-20 carbon atoms,
their tautomers, their optically active forms, including single diastereomeric forms, mixtures thereof, racemic mixtures of these compounds, and their physiologically acceptable salts of inorganic and organic acids or bases.

2. The nucleotide derivative according to claim 1, wherein $R^1$ and $R^2$ are independently a straight-chain $C_8$-$C_{16}$ alkyl group.

3. The nucleotide derivative according to claims 1 to 2, wherein $R^1$ and $R^2$ represents independently a decyl-, undecyl- or dodecyl-residues.

4. The nucleotide derivative according the claims 1-3, wherein the compound is selected from the group consisting of

- [2-Chloro-9-(2'-deoxy-2'-fluoro-β-D-arabinofuranosyl)adenine]-5'-phosphoric acid-(2-decyloxy 3-dodecyloxy) propyl ester (mixture of diastereomers)
- [2-Chloro-9-(2'-deoxy-2'-fluoro-β-D-arabinofuranosyl)adenine]-5'-phosphoric acid-(2*R*-decyloxy 3-dodecyloxy)propyl ester
- [2-Chloro-9-(2'-deoxy-2'-fluoro-β-D-arabinofuranosyl)adenine]-5'-phosphoric acid-(2*S*-decyloxy 3-dodecyloxy)propyl ester
- [2-Chloro-9-(2'-deoxy-2'-fluoro-β-D-arabinofuranosyl)adenine]-5'-phosphoric acid (2,3-didodecyloxy)propyl ester
- [2-Chloro-9-(2'-deoxy-2'-fluoro-β-D-arabinofuranosyl)adenine]-5'-phosphoric acid (2,3-didecyloxy)propyl ester

5. The nucleotide derivative according to claim 1- 4, wherein the compound is:

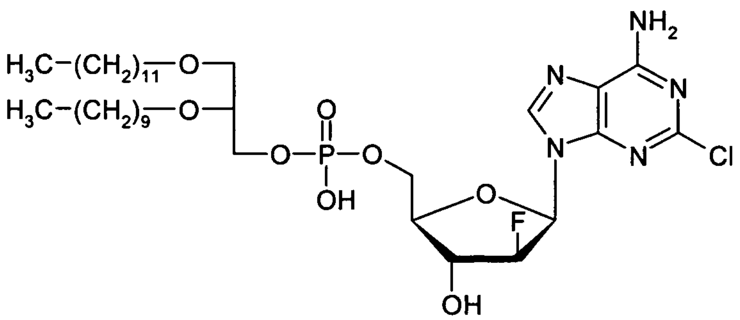

6. A pharmaceutical composition comprising at least one compound according to claims 1 - 5 in combination with a pharmaceutically acceptable adjuvant or vehicle.

7. Use of a compound according to claims 1-5 for the preparation of a medicament for treating malignat tumors, wherein said compound is to be administered to a patient in need of such treatment in an amount effective to treat said tumors.

8. The method according to claim 7, wherein said tumor is selected from the group consisting of carcinomas, sarcomas or leukemias.

9. A method of synthesis of compounds of the formula I:

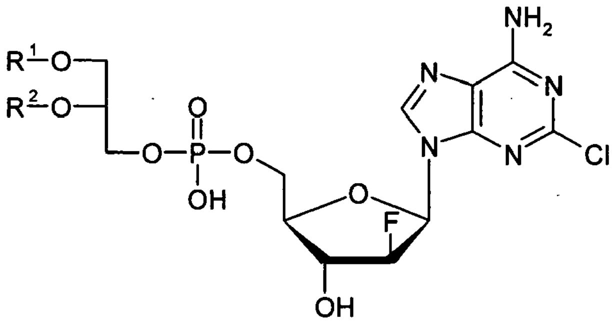

I

wherein $R^1$ and $R^2$ have the meaning of claim 1
comprising the reaction of 2-Chloro-9-(2'-deoxy-2'-fluoro-β-D-arabinofuranosyl)adenine:

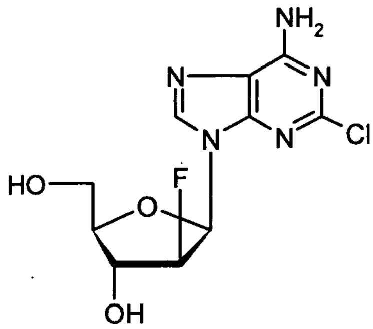

(III)

with an activated form of the compound:

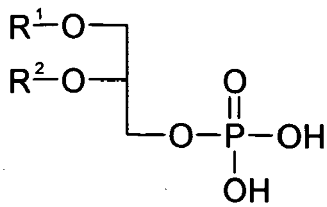

(II)

in an inert solvent to provide I: thereafter the compound is converted optionally in a physiological acceptable salt with an inorganic or organic acid or base.

European Patent Office

# EUROPEAN SEARCH REPORT

Application Number
EP 07 02 4198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | EP 1 460 082 A (HEIDELBERG PHARMA HOLDING GMBH [DE]) 22 September 2004 (2004-09-22)<br>* claims 20-30 *<br>* page 4, paragraphs 24,25 *<br>* page 6, paragraph 39 *<br>----- | 1-9 | INV.<br>C07H19/20<br>A61K31/7076 |
| A | WO 96/15234 A (BOEHRINGER MANNHEIM GMBH [DE]; HERRMANN DIETER [DE]; OPITZ HANS GEORG) 23 May 1996 (1996-05-23)<br>* page 8, line 14 - page 10, line 9 *<br>* page 15, lines 11-19 *<br>* page 16, lines 11-20 *<br>----- | 1-9 | |
| A | BERDEL W E ET AL: "CYTO TOXICITY OF THIO ETHER LYSO PHOSPHO LIPIDS IN LEUKEMIAS AND TUMORS OF HUMAN ORIGIN"<br>CANCER RESEARCH, AMERICAN ASSOCIATION FOR CANCER RESEARCH, BALTIMORE, MD, US,<br>vol. 43, no. 11,<br>1 January 1983 (1983-01-01), pages 5538-5543, XP008076329<br>ISSN: 0008-5472<br>* the whole document *<br>----- | 1-9 | TECHNICAL FIELDS SEARCHED (IPC)<br>C07H<br>A61K |

The present search report has been drawn up for all claims

| Place of search | Date of completion of the search | Examiner |
|---|---|---|
| Munich | 30 April 2008 | Gohlke, Pascale |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 07 02 4198

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2008

| Patent document cited in search report | Publication date | Patent family member(s) | Publication date |
|---|---|---|---|
| EP 1460082 A | 22-09-2004 | CN 1771253 A | 10-05-2006 |
| | | ZA 200507541 A | 31-05-2006 |
| WO 9615234 A | 23-05-1996 | AT 292173 T | 15-04-2005 |
| | | AU 711367 B2 | 14-10-1999 |
| | | AU 3927895 A | 06-06-1996 |
| | | CA 2204908 A1 | 23-05-1996 |
| | | EP 0791056 A2 | 27-08-1997 |
| | | ES 2239761 T3 | 01-10-2005 |
| | | HU 77486 A2 | 28-05-1998 |
| | | JP 10508498 T | 25-08-1998 |
| | | JP 3681005 B2 | 10-08-2005 |
| | | JP 2005095178 A | 14-04-2005 |
| | | NZ 295682 A | 29-09-1999 |
| | | PT 791056 T | 31-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0350287 A
- US 5512671 A
- WO 9203462 A
- EP 1460082 A
- WO 0160383 A
- US 6680382 B
- WO 9606620 A
- DE 3934820
- EP 0315973 A
- EP 315973 A


### Non-patent literature cited in the description


- *J. Biol. Chem.,* 1990, vol. 265, 6112-6117
- *J. Org. Chem.,* 1969, vol. 34, 2632-2636
- *Hematology,* 1999, 463
- *J. Med. Chem.,* 1992, vol. 35, 397-401